# EUROPEAN PATENT APPLICATION

(11) **EP 0 870 433 A1**
(43) Date of publication of application: **14.10.1998**
(21) Application number: 98201072.0
(22) Date of filing: 09.04.1998
(51) Int. Cl.: A23K 1/10, A61L 11/00, A22B 7/00

(54) **Apparatus and method for inactivating animal products.**

(30) Priority: 10.04.1997 NL 1005784
(71) Applicant: Dupps Europe B.V., 1703 AZ Heerhugowaard (NL)
(72) Inventor: Pathuis, Gerrit, 1701 JG Heerhugowaard (NL); Bellentani, Renato, 41051 Castelnuovo Rangone (IT)
(74) Representative: Ferguson, Alexander

(57) **Abstract**

The invention relates to an apparatus for inactivating animal products especially of ruminants. In order to carry out the inactivating in an apparatus in which the animal products are treated in a continuous process, according to the invention an elongated chamber is provided with a closable supply opening and a closable discharge opening, having therein means for continuous transport of the animal products, in which there are heating means for heating the animal products to a desired temperature, and in which there are pressure means for bringing the inside of the chamber to a desired pressure and keeping it there.

The invention also relates to a method for inactivating animal products.

## Description

The invention relates to an apparatus for inactivating animal products especially of ruminants. The invention also relates to a method for inactivating these products.

With ruminants intended for meat products about 40% of the animal is left over after the meat has been removed, which is processed into animal meal and animal fats. These remaining parts are for that purpose broken into pieces with a cross section of at most 25 by 25 mm (the length is variable) and then dehydrated in a continuous process under atmospheric pressure in fat at 140°C to a water percentage of approximately 3%. The average length of time in the fat amounts to 90 minutes. The fat is then pressed out of the dehydrated pieces and the pieces ground to animal meal. This whole process is carried out as a continuous process. In this field the processed material is called cracklings. All kinds of mammals, birds and fish can be processed in such a way.

In connection with the BSE disease (Bovine Spongiform Encephalopathy) and scrapie, the European Union has stipulated that animal meal from ruminants is inactivated by heating the animal products to be processed to animal meal for at least 20 minutes under a pressure of at least 3 bar absolute (300kPa) at at least 133°C. This inactivating step can take place on the raw material or later in the processing operation.

A suchlike inactivating step in which a minimal time span is required, can be carried out in a relatively simple way in batches, by retaining a certain quantity of cracklings, for instance, in a vessel or the like and keeping it there for the required length of time at the required temperature and pressure.

A suchlike batch processing has the disadvantage that this cannot be applied in the process as described above, in as this process would then have to be repeatedly interrupted.

It is an object of the invention to inactivate the animal products in such a way that no use has to be made of batch processing.

According to a first aspect of the invention, for that purpose an apparatus is provided for inactivating animal products especially of ruminants, comprising an elongated chamber with a closable supply opening and a closable discharge opening, having therein means for continuous transport of the animal products, in which there are heating means for heating the animal products to a desired temperature, and in which there are pressure means for bringing the inside of the chamber to a desired pressure and keeping it there.

By providing the means for continuous transport in the chamber, the animal products or cracklings are conveyed from the supply opening to the discharge opening, it being ensured that by making a suitable choice of chamber length in combination with the velocity of the conveying means, the cracklings remain for the desired period in the chamber, at the desired temperature and pressure. The conveying means ensure that no mixing of the cracklings occurs in the chamber, so that the desired length of stay in the chamber is guaranteed.

According to a preferred embodiment the means for continuous transport comprise a screw conveyor or a ribbon feeder, which is accommodated in an substantially U-shaped trough in the chamber. A screw conveyor or a ribbon feeder is a reliable construction element which takes up little space and is available on the market. A ribbon feeder which can be envisaged as a screw without a central axis has the added advantage that a larger filling of the trough is possible. Instead of a screw conveyor or ribbon feeder it is possible to use other means for continuous transport, such as a conveyor circulating in the housing with blades or spoons moving in the trough which take the cracklings along. However, a conveyor of that kind does need more space.

Preferably the heating means comprise a casing arranged against the trough, through which casing a heating medium can be led, preferably steam under high pressure and at a high temperature, so that the cracklings in the trough are heated directly through the trough by the heating means led through the casing.

Preferably the pressure means comprise a supply for steam under the desired pressure and a discharge for saturated steam, in which preferably the supplied steam is also at the desired temperature, so that the inside of the chamber can be brought to and kept at the desired pressure, in which the air/steam mixture which surrounds the cracklings immediately has the correct temperature.

According to a preferred embodiment the supply and discharge opening of the chamber are closable with the help of a respective blade valve, so that the supply and discharge opening can quickly be opened and closed.

In order that the elongated chamber can be filled while the desired pressure therein is maintained, a supply pressure vessel is provided ahead of the supply opening of the chamber, from where the animal products can be brought into the chamber, preferably with help of a screw, in which whilst introducing the cracklings into the chamber the pressure in the pressure vessel is as high as that in the chamber.

Preferably the supply pressure vessel can be filled from a supply bunker, which is connected to the supply pressure vessel by means of a closable passage, in which during filling of the pressure vessel from the supply bunker, which takes place under atmospheric pressure, the supply opening of the chamber has to be closed. Filling the pressure vessel can take place with the help of a rapid rotating screw.

So as to obtain the desired pressure in the pressure vessel, the supply pressure vessel is provided with supply and discharge conduits, respectively, so as to bring the supply pressure vessel to the desired pressure and the ambient pressure, respectively. In this way air under pressure can be supplied and the pressurized air discharged from the pressure vessel.

Preferably the supply conduit is provided with heating means, so that the cracklings do not cool off too hard in the pressure vessel.

In order to discharge the inactivated cracklings a discharge pressure vessel is provided behind the discharge opening of the chamber, into which the inactivated animal products can be introduced from the chamber, preferably with the help of screw. During introduction in the discharge pressure vessel, the pressure therein is as high as in the chamber.

Preferably the discharge pressure vessel can be emptied into a discharge bunker, which is connected to the discharge pressure vessel by means of a closable passage. Whilst emptying the discharge opening of the chamber is closed, in view of the fact that emptying takes place at atmospheric pressure. Emptying the discharge pressure vessel can take place with the help of a rapid rotating screw.

In order to attain atmospheric pressure in the discharge pressure vessel, the discharge pressure vessel is provided with discharge conduits so as to bring the discharge pressure valve to the ambient pressure. The increased pressure in the discharge vessel is caused by the supply of air/steam mixture from the chamber when the discharge opening of the chamber is open.

According to another aspect of the invention a method is provided for inactivating animal products especially of ruminants, in which the products are introduced into an elongated chamber, and in which the products are transported through the elongated chamber by means of continuous transport over a predetermined time prior to leaving the chamber, a desired temperature and a desired pressure prevailing in the chamber. With the help of this method cracklings can be inactivated as a continuous operating step in the existing continuous process as described above.

Preferably the transport time amounts to at least 20 minutes, the temperature to at least 133°C and the pressure to at least 3 bar absolute (300 kPa), so that the terms set by the European Union are met.

Preferably the animal products are supplied from a supply pressure vessel and are discharged into a discharge pressure vessel, so that no loss of pressure occurs during supply and discharge and the products are thus inactivated as quickly as possible.

Preferably the supply pressure vessel is filled from a supply bunker at an atmospheric pressure in a short period, while the chamber is cut off, the supply pressure vessel is subsequently pressurized prior to introducing the products into the chamber from the supply pressure vessel, and the discharge pressure vessel is preferably emptied at atmospheric pressure in a short period into a discharge bunker while the chamber is cut off, so that the time in which no products can be introduced into the chamber or can be discharged out of the chamber is as short as possible.

The invention will now be elucidated on the basis of an exemplary embodiment, referring to the accompanying drawing.

Figure 1 shows the whole apparatus 1 according to the invention with a perspective view of the inactivating apparatus according to the invention in a schematic fashion.

Figure 2 shows a longitudinal cross section of the apparatus according to fig. 1, in which the components are show in one plane.

Figure 2a shows a cross section of the apparatus shown in figure 2.

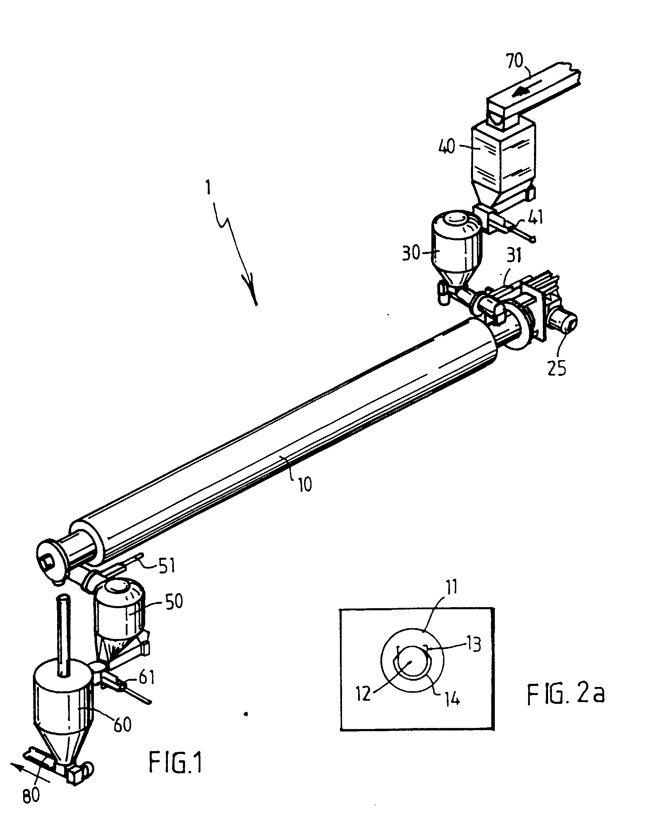

Figure 1 shows the actual inactivating apparatus 10 in which a screw conveyor is driven by a drive 25. Cracklings are introduced into the inactivating apparatus 10 from a supply pressure vessel 30, which in turn is periodically filled from a supply bunker 40. There the cracklings are continuously fed by a supply pipe 70. The inactivating apparatus 10, the supply pressure vessel 30 and the supply bunker 40 can be mutually closed with the help of blade valves 31 and 41. A discharge pressure vessel 50 is placed at the output side of the inactivating apparatus 10, which discharge pressure vessel 50 is periodically emptied into a discharge bunker 60, from where the cracklings are continuously discharged by the discharge tube 80. The inactivating apparatus 10, the discharge pressure vessel 50 and the discharge bunker 60 can be each separated from one another with the help of the blade valves 51 and 61.

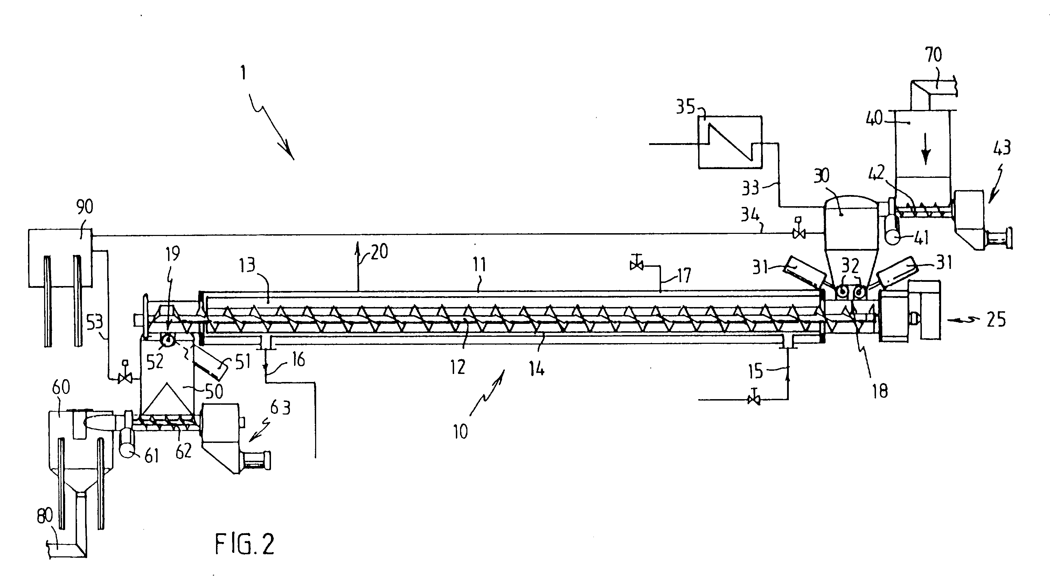

Figure 2 shows the whole apparatus 1 in one plane. The actual inactivating apparatus 10 consists of a chamber 11, in which a screw conveyor 12 is incorporated passing through a U-shaped trough 13. On the outside of the U-shaped trough 13 a casing 14 is arranged, through which steam under high pressure and at a high temperature can be led and via an inlet 15 and an outlet 16. Figure 2a shows a cross section through the chamber 11, the screw conveyor 12, the U-shaped trough 13 and the casing 14. In order to bring the inside of the chamber 11 to the desired pressure and to the desired temperature, steam can be supplied via inlet 17. So that the water content of the cracklings which are to be inactivated does not get too high, an outlet 20 for saturated steam is provided. The screw conveyor 12 is driven by a drive 25, which consists in the usual manner of a motor and a reduction gear unit.

The chamber 11 has a supply opening 18 to which a supply pressure vessel 30 is connected. Cracklings in the pressure vessel 30 can be introduced via the supply opening 18 into the chamber 11 with the help of a screw 32. The supply from the pressure vessel 30 to the supply opening 18 can be closed in transport direction behind the screw 32 with the help of a blade valve 31. In the exemplary embodiment shown, two screws 32 and two blade valves 31 are provided. The supply pressure vessel 30 can be pressurized with gas via a supply 33, which gas can be warmed with the help of a heat exchanger 35. The supply pressure vessel 30 can be brought to atmospheric pressure by discharging gas via an outlet 34 to a pressure relieving device 90.

The supply pressure vessel 30 is periodically filled from a supply bunker 40. The cracklings in the supply bunker 40 are introduced into the supply pressure vessel 30 with the help of a screw 42, which in a short time introduces the cracklings into the supply pressure vessel 30. The supply bunker 40 and the supply pressure vessel 30 can be mutually closed by means of blade valve 41.

The chamber 11 has a discharge opening 19 on the discharge side, from where the inactivated cracklings can be introduced into a discharge pressure vessel 50 with the help of a screw 52, in which the discharge opening 19 can be closed in transport direction ahead of the screw 52 with the help of a blade valve 51. Gas in the pressurized discharge pressure vessel 50 can be discharged via a discharge 53 to the pressure relieving device 90.

Periodically the inactivated cracklings can be introduced into a discharge bunker 60 from the discharge pressure vessel 50 with the help of a screw 62, which introduces the cracklings quickly into the discharge pressure bunker 60. The discharge pressure vessel 50 and the discharge bunker 60 can be mutually closed by means of the blade valve 61.

The method for inactivating cracklings will now be elucidated on the basis of figure 2.

The cracklings are continuously fed by supply tube 70. They are poured into supply bunker 40, which, with the help of the screw 42 driven by drive 43, is periodically emptied in the supply pressure vessel 30. Prior to the emptying of the supply bunker 40, the screws 32 are switched off and the supply opening 18 of the chamber 11 is closed with the help of the blade valves 31, after which the volume of the supply pressure vessel 30 is brought to atmospherical pressure by discharging gas in the supply pressure vessel 30 via the discharge 34 to the pressure relieving device 90. After the supply pressure vessel 30 is at atmospheric pressure, the passage between the supply bunker 40 and the supply pressure vessel 30, which was closed by blade valve 41, is opened, after which the contents of the supply bunker 40 are in a short time introduced into the supply pressure vessel 30 with the help of the screw 42. The passage is subsequently closed again by the blade valve 41 and the supply pressure vessel 30 is brought under pressure by gas via supply 33. After the supply pressure vessel 30 has again been brought to the desired pressure, the supply opening 18 is opened again and the screws 32 are started again to introduce the contents of the supply pressure vessel 30 into the chamber 11.

The cracklings introduced into the chamber 11 through the supply opening 18 arrive in a U-shaped trough or groove, in which the screw conveyor 12 rotates slowly, driven by the drive 25. The length of the screw 12 and the speed of revolution of the screw are selected such, that the cracklings introduced via the inlet opening 18 only arrive at the discharge opening 19 after the desired time. During the transport of the cracklings through the chamber 11 the cracklings are heated by means of the heating casing 14, through which steam under high pressure and at a high temperature is led. Likewise the contents of the chamber 11 are kept at the desired temperature and pressure by supplying steam of the desired temperature and pressure via supply 17, while saturated steam can be discharged via discharge 20, so that the water percentage of the cracklings does not exceed the desired percentage of 3%.

At the discharge opening 19 of the chamber 11 the inactivated cracklings are led into the discharge pressure vessel 50 with the help of the screw 52. The contents of the discharge pressure vessel 50 are periodically emptied in the discharge bunker 60. For that purpose the discharge opening 19 is first of all closed with the help of the blade valve 51, after which the discharge pressure vessel 50 is brought to atmospheric pressure by discharging gas via discharge 53 to pressure relieving device 90. Then the passage between the discharge pressure vessel 50 and the discharge bunker 60 which was closed with the help of the blade valve 61 is opened, after which the screw 62 in a short time empties the contents of the discharge pressure vessel 50 into the discharge bunker 60. Subsequently, the passage is closed again with the help of blade valve 61, whereupon the discharge opening 19 of the chamber 11 is opened again and the discharge cycle is repeated. From the discharge bunker 60 the inactivated cracklings are continuously discharged via the discharge tube 80 to a cooler (not shown), as the cracklings have too high a temperature because they have been inactivated to be able to be further processed directly. After cooling the cracklings are crushed or pulverized and subsequently processed in the normal manner.

In order to meet the requirements, steam at a pressure of 10 bar and a temperature of approximately 170°C is led through casing 14, so that the cracklings introduced into the chamber 11 via the supply opening 18 reach the required temperature of 133°C as quickly as possible. The passage time of the cracklings through the U-shaped trough over that portion of the trough along which the casing 14 is arranged comes to at least 20 minutes. Steam is introduced into the chamber 11 via supply 17 at a pressure of over 3 atmosphere (300 kPa) absolute, or 2 atmosphere (200 kPa) excess pressure, at a temperature of at least 133°C.

In the entire processing unit in which apparatus 1 according to the invention is incorporated, the cracklings supplied by the supply tube 70 are at a temperature of approximately 80°C. So that the cracklings that are supplied to the chamber 11 via the supply opening 18 have as high a temperature as possible, the gas that is supplied to the pressure vessel 30 is preheated with the help of heat exchanger 35. In the exemplary embodiment the chamber 11 has a length of approximately 8 metres and the cracklings are transported in a period of approximately 25 minutes from the supply opening 18 to the discharge opening 19.

Instead of a screw conveyor for transporting the cracklings, other conveying means are possible in the chamber 11, such as a ribbon feeder, in other words a metal strip wound as a screw which runs through the trough, or a circular conveyor with blades which move the cracklings through the U-shaped trough.

## Claims

1. Apparatus for inactivating animal products especially of ruminants, comprising an elongated chamber with a closable supply opening and a closable discharge opening, having therein means for continuous transport of the animal products, in which there are heating means for heating the animal products to a desired temperature, and in which there are pressure means for bringing the inside of the chamber to a desired pressure and keeping it there.

2. Apparatus according to claim 1, in which the means for continuous transport comprise a screw conveyor or a ribbon feeder, which is accommodated in a substantially U-shaped trough in the chamber, in which preferably the heating means comprise a casing arranged against the trough, through which casing a heating medium can be led, preferably steam under high pressure and at a high temperature.

3. Apparatus according to claim 1 or 2, in which the pressure means comprise a supply for steam under the desired pressure and a discharge for saturated steam, in which preferably the supplied steam is also at the desired temperature.

4. Apparatus according to any one of the preceding claims, in which the supply and the discharge opening of the chamber are closable with the help of a respective blade valve.

5. Apparatus according to any one of the preceding claims, in which a supply pressure vessel is provided ahead of the supply opening of the chamber, from where the animal products can be brought into the chamber, preferably with help of a screw, in which preferably the supply pressure vessel can be filled from a supply bunker, which is connected to the supply pressure vessel by means of a closable passage.

6. Apparatus according to claim 5, in which the supply pressure vessel is provided with supply and discharge conduits, respectively, so as to bring the supply pressure vessel to the desired pressure and the ambient pressure, respectively, in which preferably the supply conduit is provided with heating means.

7. Apparatus according to any one of the preceding claims, in which a discharge pressure vessel is provided behind the discharge opening of the chamber, into which the inactivated animal products can be introduced from the chamber, preferably with the help of screw.

8. Apparatus according to claim 7, in which the discharge pressure vessel can be emptied into a discharge bunker, which is connected to the discharge pressure vessel by means of a closable passage, in which preferably the discharge pressure vessel is provided with discharge conduits so as to bring the discharge pressure valve to the ambient pressure.

9. Method for inactivating animal products especially of ruminants, in which the products are introduced into an elongated chamber, and in which the products are transported through the elongated chamber by means of continuous transport over a predetermined time prior to leaving the chamber, a desired temperature and a desired pressure prevailing in the chamber, in which preferably the transport time amounts to at least 20 minutes, the temperature to at least 133°C and the pressure to at least 3 bar absolute (300 kPa).

10. Method according to claims 9, in which the animal products are supplied from a supply pressure vessel and are discharged into a discharge pressure vessel, in which preferably the supply pressure vessel is filled from a supply bunker at an atmospheric pressure in a short period, while the chamber is cut off, the supply pressure vessel is subsequently pressurized prior to introducing the products into the chamber from the supply pressure vessel, and the discharge pressure vessel is preferably emptied at atmospheric pressure in a short period into a discharge bunker while the chamber is cut off.
